# EUROPEAN PATENT SPECIFICATION

(11) **EP 2 258 310 B1**
(45) Date of publication and mention of the grant of the patent: **09.10.2019**
(21) Application number: 10178672.1
(22) Date of filing: 22.08.2007
(51) Int. Cl.: A61F 2/01

(54) **Vein filter**
Filter für die Vene
Filtre pour veine

(30) Priority: 29.08.2006 US 840888 P
(43) Date of publication of application: 08.12.2010
(62) Divisional of application: 07016426.4
(73) Proprietor: Argon Medical Devices, Inc., Plano, TX 75024 (US)
(72) Inventor: McGuckin Jr., James F, Radnor, PA 19087 (US); Bressler, James E., Langhorne, PA 19047 (US); Schaller, David M., Wallingford, PA 19086 (US)
(74) Representative: Lavoix

(56) References cited:
- EP-A1- 1 103 233
- EP-A1- 1 616 530
- US-A1- 2006 079 928

## Description

This application relates to a vascular filter and more particularly to a vein filter for capturing blood clots within the vessel.

Passage of blood clots to the lungs is known as pulmonary embolism. These clots typically originate in the veins of the lower limbs and can migrate through the vascular system to the lungs where they can obstruct blood flow and therefore interfere with oxygenation of the blood. Pulmonary embolisms can also cause shock and even death.

In some instances, blood thinning medication, e.g. anticoagulants such as Heparin, or sodium warfarin can be given to the patient. These medications, however, have limited use since they may not be able to be administered to patients after surgery or stroke or given to patients with high risk of internal bleeding. Also, this medication approach is not always effective in preventing recurring blood clots.

Therefore, surgical methods to reduce the likelihood of such pulmonary embolisms by actually blocking the blood clot from reaching the lungs have been developed. To this end, minimally invasive surgical techniques have been developed involving the placement of a mechanical barrier in the inferior vena cava. These barriers are in the form of filters and are typically inserted through either the femoral vein in the patient's leg or the right jugular vein in the patient's neck or arm under local anesthesia. The filters are then advanced intravascularly to the inferior vena cava where they are expanded to block migration of the blood clots from the lower portion of the body to the heart and lungs.

These prior filters take various forms. One type of filter is composed of coiled wires such as disclosed in U.S. Patent Nos. 5,893,869 and 6,059,825. Another type of filter consists of legs with free ends having anchors for embedding in the vessel wall to hold the filter. These filters are disclosed, for example, in U.S. Patent Nos. 4,688,553, 4,781,173, 4,832,055, and 5,059,205, 5,984,947 and 6,007,558. Another type of filter is disclosed in U.S. Patent no. 6,214,025 consisting of wires twisted together to form a cylindrical anchoring portion conforming to the inner vessel wall surface to exert a radial force and a conical filtering portion.

Several factors have to be considered in designing vein filters. One factor is that the filter needs to be securely anchored within the vessel wall, while avoiding traumatic engagement and damage to the wall as well as damage to the neighboring abdominal aorta. Another factor is that the filter must be collapsible to a sufficiently small size to be easily maneuvered and atraumatically advanced intravascularly to the inferior vena cava or other target vessel. Thirdly, the filter should direct the blood clots to the center of the vessel to improve dissolution of the clot within the vessel by the blood flow.

US-A-2006079928 discloses a permanent blood clot filter which may be retrieved. The blood clot filter includes a filter section and an alignment section. The filter section includes a filter hub and a set of filter legs whose downstream ends are connected to the filter hub. The filter legs extend axially and radially outwardly from the filter hub to form a conical configuration. The alignment section includes an alignment hub and a set of alignment ribs whose downstream ends are connected to the alignment hub and whose upstream ends are connected to the filter legs. The alignment ribs extend from the alignment hub radially outwardly and then further extend radially inwardly to provide centering of the filter.

The filters disclosed in the commonly assigned co-pending application 10/889,429 (hereinafter "the '429 application") (EP-A-1 616 530), satisfy the foregoing parameters. The filters have sufficient anchoring force to retain the filter within the vessel while providing atraumatic contact with the vessel wall, have a minimized insertion (collapsed) profile to facilitate delivery through the vascular system to the surgical site, and direct migration of the captured blood clots to the center of the vessel. The filters also provide simplified insertion through the femoral or the right jugular vein or arm into the inferior vena cava.

The filters of the '429 application can advantageously be readily removed minimally invasively, e.g. intravascularly, from the patient, thus advantageously providing for a temporary filter. Thus, these filters advantageously strike the balance of having structure to provide sufficient anchoring while enabling atraumatic removal from the vessel after a period of time. Certain filters of the '429 application also advantageously have a retrieval end configured to facilitate grasping by a snare as well as to facilitate withdrawal by providing a smooth transition into a retrieval sheath.

The filters of the '429 are very effective in achieving their desired functions, whether used as a permanent or temporary filter. It is an object of the present application to provide a modification to the filters to even further facilitate removal if used as a temporary filter.

The filters of the '429 application also have effective retention hooks to grasp the vessel wall to prevent migration of the filter. It is an object of the present application to provide an alternative retention hook to even further enhance retention.

According to the present invention there is provided a vessel filter as disclosed in the appended claims, comprising a body made from a single tube, the tube cut to create a plurality of elongated struts forming a filter region and a mounting region having a greater transverse dimension than a transverse dimension of the filter region, the mounting region including a plurality of the elongated struts which terminate in vessel engaging hooks, the hooks having a penetrating tip pointing in a direction toward the filter region, a plurality of teeth for engaging the vessel, and a heel extending beyond the teeth in a direction opposite the direction of the penetrating tip, wherein in a collapsed configuration of the filter the hooks extend substantially perpendicular to the respective strut and in the plane thereof whereby the penetrating tip points in a first direction toward a cranial end of the filter and the teeth extend in a second direction substantially in a longitudinal axial direction of the filter so as to point toward the caudal end of the filter.

The teeth may be provided solely on the vessel engaging hooks which extend substantially perpendicular to the respective strut.

The teeth may be provided on a convexly curved surface of the vessel engaging hooks.

An end of the penetrating tip may extend substantially parallel to the longitudinal axis of the strut.

The heel of the hooks may extend at an angle to the longitudinal axis of the respective struts in the mounting region.

The heels of adjacent vessel engaging hooks may terminate axially spaced.

The hooks may have a curved end surface.

The penetrating tip may extend in a first direction toward the filter region and the heel may extend in a second opposite direction.

The vessel filter may comprise a first region and a second region, the filter movable between a collapsed position for delivery to the vessel and an expanded position for placement within the vessel, the first region having a filter portion having a converging region to direct particles toward the center of the filter, the first region including a plurality of spaced apart filter struts, the struts each having a strut width defined as a distance between a first wall and a second wall, and the strut having a longitudinal axis, a plurality of hooks at the second region, the hooks having a vessel penetrating tip, the hooks positioned on a distal end portion of the strut, and having a width greater than the width of the strut from which it extends radially such that the penetrating tip of the hook extends beyond the first wall. The struts may each have a first width defined as a distance between the first wall and the second wall and having a longitudinal axis, and the hook may have a third and fourth wall and a second width defined between the first and second walls, the width of the hook being greater than the first width of the strut from which it extends such that the penetrating tip portion of the hook extends radially beyond the first wall. The heel may extend radially beyond the second wall of the respective strut.

The strut may have a reduced diameter area transitioning into the hook, the reduced area providing a space to accommodate the heel of an adjacent hook. The heel of the hooks may have a third width less than the first width of the respective strut.

The vessel filter may further comprise connecting filter struts extending at an angle from the filter struts to join adjacent filter struts.

The filter may be formed from a laser cut tube and composed of shape memory material.

The vessel filter may further comprise a retrieval hook, the retrieval hook having a cutout exposing an internal annular surface, the annular surface dimensioned to receive a portion of a retrieval sheath.

Preferred embodiment(s) of the present disclosure are described herein with reference to the drawings wherein:
Figure 1 is a perspective view of a first embodiment of a vein filter of the present invention in the collapsed (retracted) configuration, and shown removed from a delivery tube/sheath;
Figure 2 is an enlarged broken side view of a portion of the vein filter of Figure 1;
Figure 3 is a developed view of the retention hooks of the vein filter of Fig. 1;
Figure 4A is a perspective view of the vein filter of Figure 1 in an expanded (radially extending) configuration;
Figure 4B is a side view of the vein filter of Figure 4A;
Figure 5 is a front view of the vein filter of Figure 4A;
Figure 6 is a side view of the vein filter of Figure 1 with the struts in the expanded configuration and the spacers in the collapsed configuration;
Figure 7 is a close up perspective view of the detail of Figure 6;
Figure 8 is a perspective view of a cranial end of a filter of the '429 application showing the retrieval hook of the filter;
Figure 9 is a view similar to Figure 8 except showing the cranial end of the filter of Figure 1, the spacers shown in the collapsed position;
Figure 9A is a perspective view of an alternate embodiment of the retrieval portion of the filter having an extended hook;
Figure 10 is a view similar to Figure 9, except showing a broken view of the spacers extending radially from the tubular portion;
Figure 11 is a perspective view of an alternate embodiment of a vein filter of the present invention having a single spacer loop, the filter and spacer shown in the expanded configuration;
Figure 12 is a front view of the filter of Figure 11;
Figures 13, 14, and 15 illustrate delivery and placement of the vessel filter of Figure 1 in the inferior vena cava wherein Figure 13 illustrates initial insertion of the delivery sheath through the femoral vein, Figure 14 illustrates the delivery sheath being advanced toward the inferior vena cava just below (upstream) the juncture of the renal arteries; and Figure 15 illustrates the filter in the expanded placement configuration in the inferior vena cava;
Figure 15A illustrates an initial step in removal of the filter from the inferior vena cava by a retrieval snare and catheter;
Figure 16 is a side view of an alternate embodiment of the filter of the present invention having spacers at different angles to the longitudinal axis of the filter, the spacers shown in the expanded position;
Figure 17 is a perspective view of an alternate embodiment of the filter of the present invention having a single spacer extending in a single plane, the spacers shown in the expanded position;
Figure 17A is a close up view of the area of detail of Figure 17;
Figure 18 is a perspective view of another alternate embodiment of a filter of the present invention having a single spacer extending in multiple planes;
Figure 19 is a perspective view of the cranial end of another alternate embodiment of a filter of the present invention having two spacers, the spacers shown in the expanded position;
Figure 19A is a view similar to Figure 19 except showing the spacers in the collapsed position;
Figure 20 is a perspective view of the cranial end of yet another alternate embodiment of a filter of the present invention having two spacers, the spacers shown in the expanded position;
Figure 20A is a view similar to Figure 20 except showing the spacers in the collapsed position;
Figure 21 is a perspective view of the cranial end of another alternate embodiment of a filter of the present invention showing the two spacers in the expanded position;
Figures 22A-22C illustrate another alternate embodiment of the cranial end of a filter of the present invention wherein Figure 22A is a perspective view of the cranial end in the collapsed configuration, Figure 22B is a side view in the collapsed configuration and Figure 22C is a perspective view in the expanded configuration.

Turning now to the drawings, wherein like reference numerals identify similar or like components throughout the several views, the vein filters of the present invention are described for placement within the inferior vena cava to capture blood clots or other particles which could otherwise pass to the lungs.

The filter is movable from a low profile collapsed configuration to facilitate insertion through the delivery sheath to a larger expanded placement configuration to enable atraumatic engagement with the vessel walls to secure (mount) the filter within the inferior vena cava. The filter is preferably substantially bell-shaped and preferably has a flared or mounting region (portion/section) and a filtering region (portion/section). The filtering region has inwardly directed struts, terminating in a converging region, thereby directing particles toward the central axis of the filter. By directing the particles to the center, they will be exposed to greater blood flow (since there is greater flow at the center than near the wall of the vessel) which improves dissolution of the particles. The filter increases in transverse dimension to form a flared region. The flare provides less contact area than a straight region, resulting in less tissue ingrowth to facilitate removal of the filter if desired. The flare also reduces the chance of vessel distortion if inserted into a curved vena cava. The filter also has spacers to space the cranial end of the filter from the vessel wall to facilitate removal.

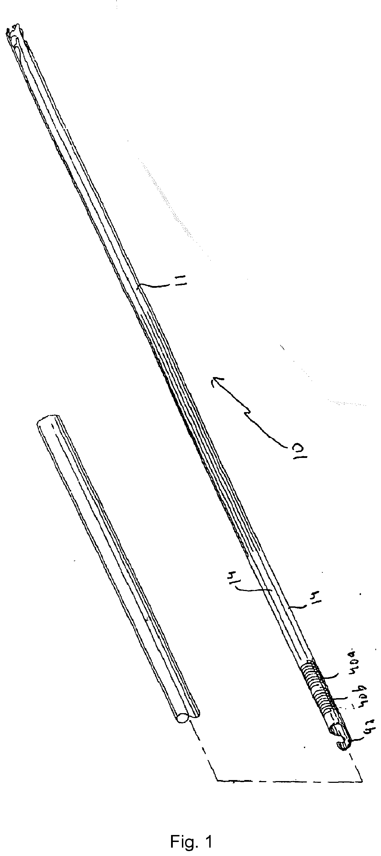

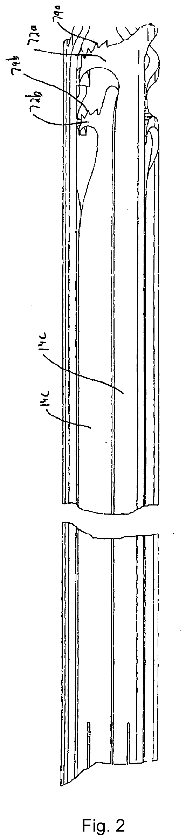

Turning now to details of the filter of the present invention and with initial reference to Figures 1 and 2, the filter is designated generally by reference numeral 10 and is shown in a collapsed configuration for delivery. Filter 10 is preferably formed from a single tube 11. In a preferred embodiment, the filter tube 11 is composed of shape memory material, such as Nitinol, a nickel titanium alloy, or elgiloy, however, other materials such as stainless steel are also contemplated. A plurality of cutouts are formed in the filter 10, preferably by laser cutting although other techniques are contemplated. In the illustrated embodiment, six elongated cutouts are formed, creating six strips or struts 14 of substantially uniform width separated by the cutouts.

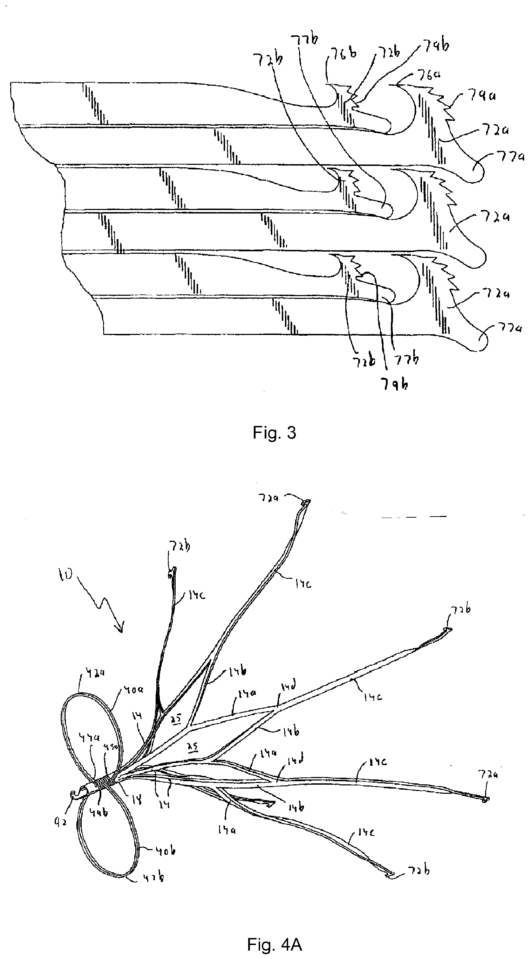

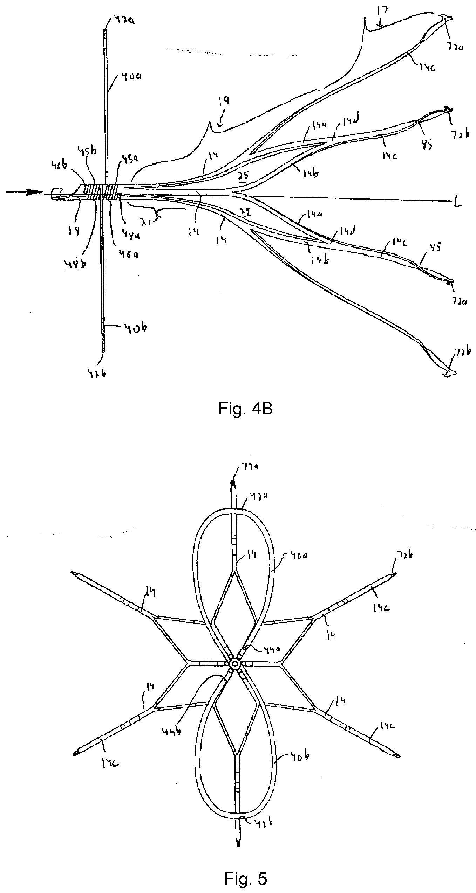

The collapsed configuration of filter 10 reduces the overall profile to facilitate delivery to the site. The diameter or transverse dimension of filter 10 in the collapsed configuration is preferably about 2mm and more preferably about 1.7mm. Other dimensions are also contemplated. The filter is thus preferably dimensioned for insertion through a 6 French delivery system and through a 6 French catheter. The diameter or transverse dimensions of the filter in the expanded placement configurations (e.g. Figs. 4A and 4B) is greater than the diameter or transverse dimension in the collapsed (delivery) configuration of Figure 1.

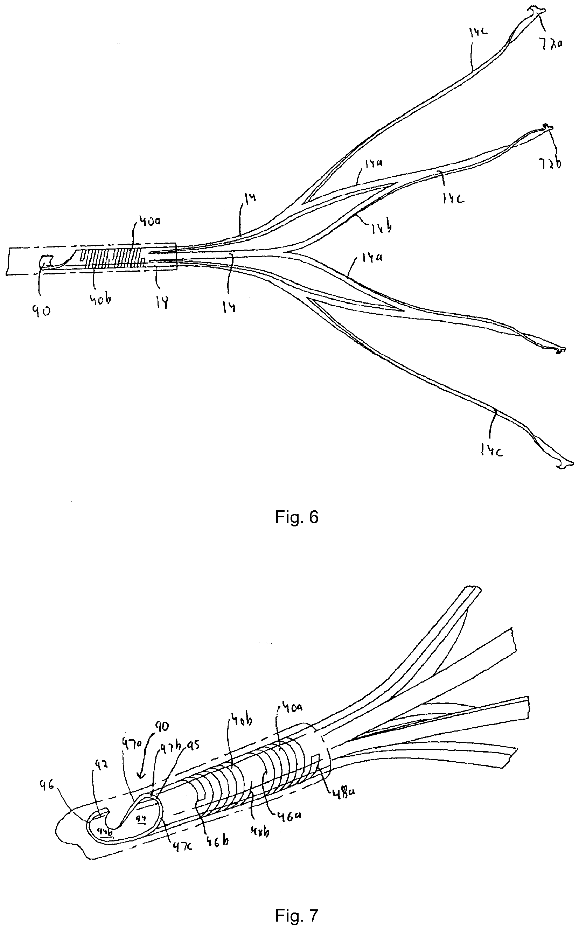

Figures 4-5 illustrate the expanded placement configuration of the filter 10. Figures 6 and 7 illustrate the expanded configuration of the struts with the spacers in the collapsed position (not exposed from the sheath) to help illustrate the invention, a configuration that would occur briefly. Filter 10 is generally bell-shaped in configuration. Filter 10 has a flared region 17 and a converging region 21 at the filtering section 19. The transverse dimension of the filter at the flared (or mounting/anchoring) region 17 is greater than the transverse dimension at filtering section 19. Diameters (or transverse dimensions) preferably range from about 18mm to about 32mm, depending on the internal diameter of the vessel wall as will be explained in more detail below. Other dimensions are also contemplated. The elongated struts 14 are spaced apart as shown and extend at an angle away from the longitudinal axis L of filter 10 in region 17 to provide a flare. Preferably, this angle or taper is about 8°, although other dimensions are contemplated. When expanded, the six struts 14, as shown, are preferably spaced approximately 60 degrees apart. It is also contemplated that a fewer or greater number of struts and spacing other than 60 degrees be provided.

Filtering section 19 extends from the flared region 17, and extends toward the central longitudinal axis L of the filter 10 and converges into tubular portion 18 at the cranial end of the filter.

The struts 14 of filter 10 terminate in hooks 72a, 72b which extend substantially perpendicular from the strut, achieved by torquing the struts at the region 85 so the hooks bend out of the plane. A first set of hooks 72a is larger than a second set of hooks 72b. Preferably when formed in a laser cut tube, hooks 72a are formed so that they occupy a region equivalent to the transverse dimension of two adjacent struts. Smaller hooks 72b are spaced axially with respect to each other and axially inwardly with respect to larger hooks 72a as in the filter hooks of the '429 application to minimize the collapsed profile (transverse dimension) of the filter when collapsed for insertion. The penetrating tips 76a, 76b of hooks 72a, 72b, respectively, penetrate the tissue to retain the filter, preferably temporarily, and point distally, toward the cranial (or distal) end of the filter.

Each of the hooks 72a, 72b has a series of teeth 79a, 79b, respectively to engage the vessel wall to provide additional retention to prevent movement of the filter in the caudal direction. In a preferred embodiment, the larger hooks 72a have four teeth and the smaller hooks 72b have three teeth, although a different number of teeth could be provided. A heel 77a, 77b, is provided which extends past (proximally or caudal of) the respective hook 72a, 72b to function as a stop to prevent the filter strut portions from going through the vessel wall. The angle of the heel 77b in the smaller hooks 72b is less than the angle in the larger hooks 72a to provide room for nesting of the hooks as shown in Figure 3. For clarity, not all of the hooks are fully labeled. Note this hook configuration with the teeth and/or heel can be utilized with the filters of the '429 application.

The six filter struts or strut portions 14 curve outwardly from tubular portion 18, extend radially therefrom and divide into two connecting filter struts or strut portions 14a, 14b (preferably of equal width, although differing dimensions are contemplated) that angle way from each other (in different directions) to extend to the connecting strut portion of an adjacent strut 14. Thus, connecting strut portion 14a of one strut 14 interconnects with the connecting strut portion 14b of an adjacent strut at joining region 14d. This forms closed geometric shapes 25, preferably substantially diamond shaped in configuration. For clarity, not all of the identical parts are labeled in the drawing.

In the illustrated embodiment, preferably six struts are provided forming twelve interconnecting struts, however a different number of struts and closed geometric shapes can be provided. Note that, although all six struts 14 are shown interconnected, it is also contemplated that fewer than all the struts can be interconnected. Also, the strut width can vary as described with respect to the filters disclosed in the '429 application.

After convergence of strut portions 14a, 14b at joining region 14d, it transitions into elongated mounting strut portions 14c which form flared mounting or anchoring region 17. The length of the strut portions 14c in the anchoring region 19 can vary, with increased/decreased length increasing the flexibility/rigidity of the struts. The thickness of the strut portions can also vary to affect flexibility/rigidity.

As in the other embodiments described in the '429 applications, terms such as interconnected, joined, etc., are used for ease of description, it being understood that preferably these portions are integral as they are preferably formed from a single tube. Also, mounting struts and filter struts used to describe the various embodiments disclosed herein can be considered as mounting strut "portions" or "sections" and filter strut "portions" or "sections" of the same struts if the filter is formed integrally, e.g. from a cut tube.

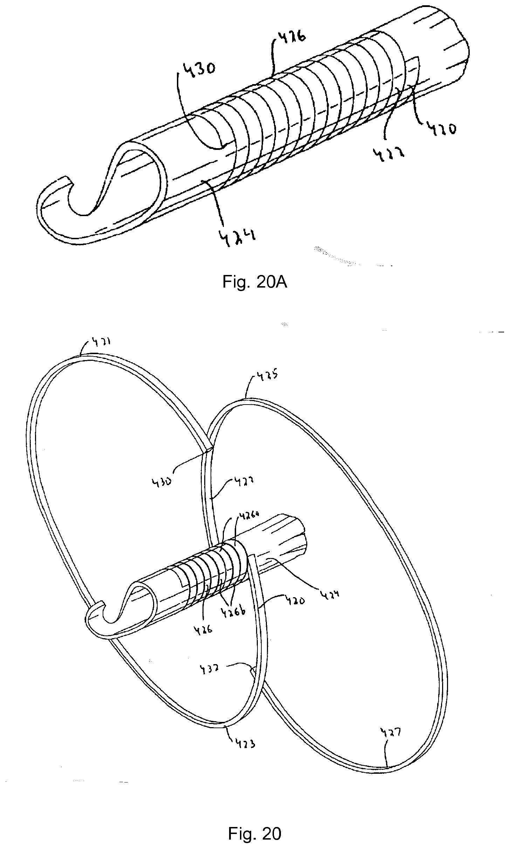

The tubular portion 18 is preferably in the form of a retrieval hook 92 as described with respect to the embodiment of Figure 20 in the '429 application. Other retrieval structure can also be utilized. Hook 92 is described in more detail below.

Two spiral cuts 45a, 45b are formed in the tube during manufacture, preferably by laser cutting, to enable two strips to be formed creating first and second spacers 40a, 40b for the filter. In the collapsed position, spacers 40a, 40b are in a substantially aligned position with respect to tubular portion 18, i.e. substantially flush with the tubular portion 18. Spacers 40a, 40b are maintained in this collapsed position during delivery to the surgical site. (see e.g. Figure 1). The spacers 40a, 40b have a shape memorized position forming loops as shown in Figure 4A. Thus, once exposed from the delivery sheath, the spacers 40a, 40b move from their collapsed position to their shape memory looped position of Figures 4A, 4B and 5. The surface 42a, 42b of the loop of each spacer 40a, 40b engages opposite sides (lying approximately 180° apart) of the vessel wall to maintain centering of the cranial end of the filter and to space tubular portion 18 and retrieval hook 92 away from the vessel wall. This spacing prevents tissue ingrowth around the hook, thereby making it easier to grasp and remove filter 10.

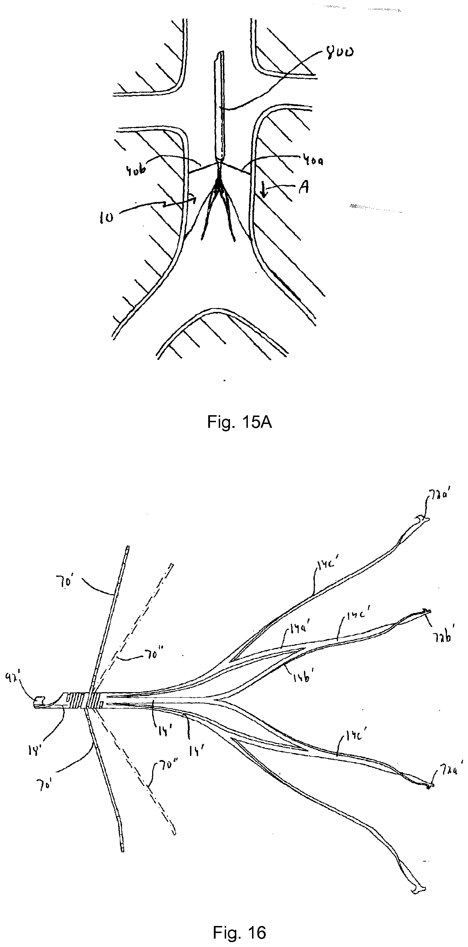

The loops of spacers 40a, 40b are open, somewhat oval shaped loops, terminate in ends 44a, 44b and lie in substantially alternate spiral planes. The first strip cut into tubular portion 18 unravels from a proximal end 48a of cutout 45a to a distal end 46a of cutout 45a to form spiral spacer 40a (see e.g. Figures 4B and 7). The second strip cut into tubular portion 18 unravels from a proximal end 48b of cutout 45b to a distal end 46b of cutout 45b to form spiral spacer 40b. In the Figure 4 embodiment, the spacer loops 40a, 40b lie in planes that are substantially perpendicular to the longitudinal axis L of the tubular portion 18 and filter 10. However, alternatively the spacer loops could lie in planes at angles other than 90 degrees and could lie in planes not parallel to each other. Examples of different angles of spacer loops with respect to the longitudinal axis of the tubular portion of the filter are shown by way of example in Figure 16. Two angled spacer loops (e.g. about 75 degrees) are designated by reference numeral 70' and smaller acute angled spacer loops, (e.g. about 45 degrees) are designated in phantom by reference numeral loop 70". The other components of the filter are identical to filter 10 and are designated with corresponding prime (') reference numerals.

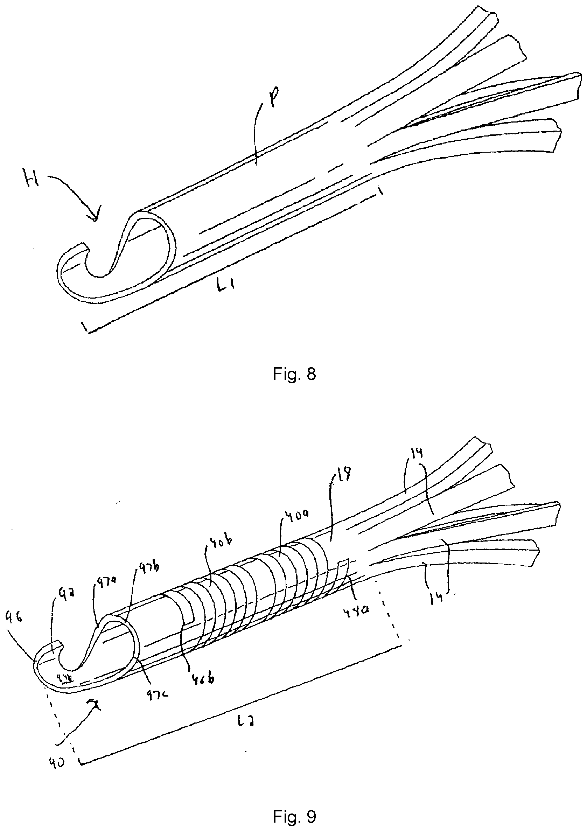

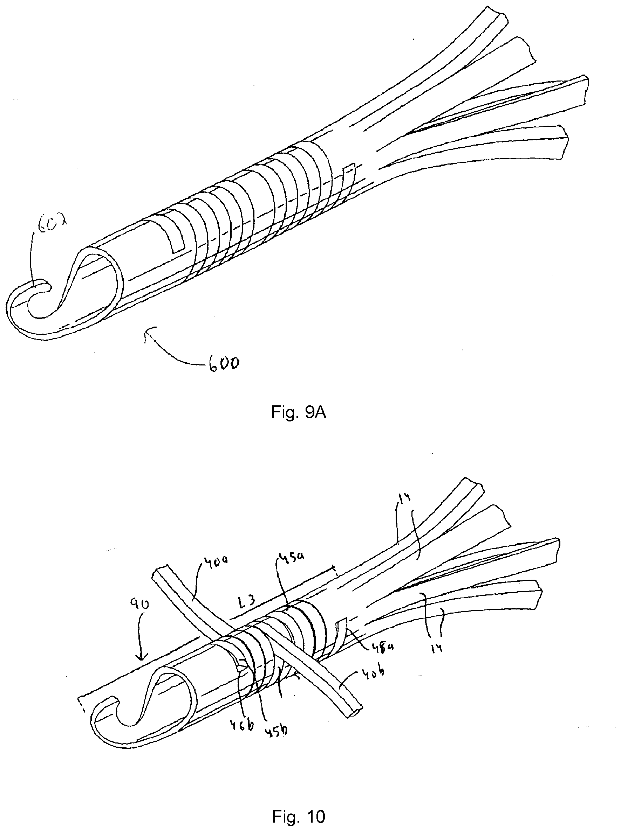

A comparison of Figures 8-10 illustrates that in the preferred embodiment, the length of tubular portion 18 remains substantially unchanged once the filter is implanted, even with the addition of the spacers. Figure 8 illustrates a cranial end of a filter of the '429 application showing the retrieval hook H of the filter. The tubular portion P has a length L1 preferably ranging from about 2.54 mm (about .100 inches) to about 15.24 mm (about .600 inches), and preferably about 7.62 mm (about .300 inches). The tubular portion 18 of Figure 9, which is the embodiment of Figure 1, has a length L2, preferably ranging from about 12.7 mm (about .500 inches) to about 43.18 mm (about 1.700 inches), and preferably about 22.38 mm (about .881 inches) which is greater than length L1 due to the space needed to create the spiral spacers 40a, 40b. However, once the spacers 40a, 40b move from their aligned position to their expanded position, the end of the tubular portion 18 contracts to close the gap created by the spiral cutouts to move to a length L3 which is preferably closed to length L1.

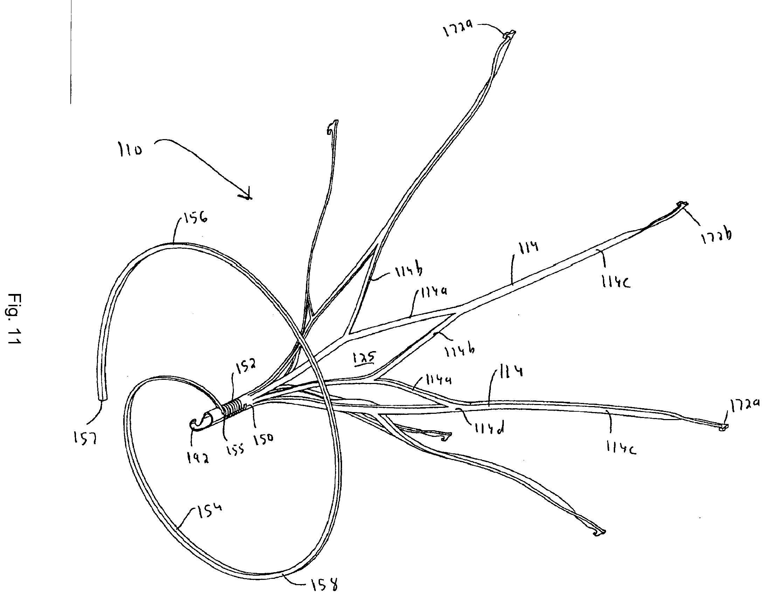

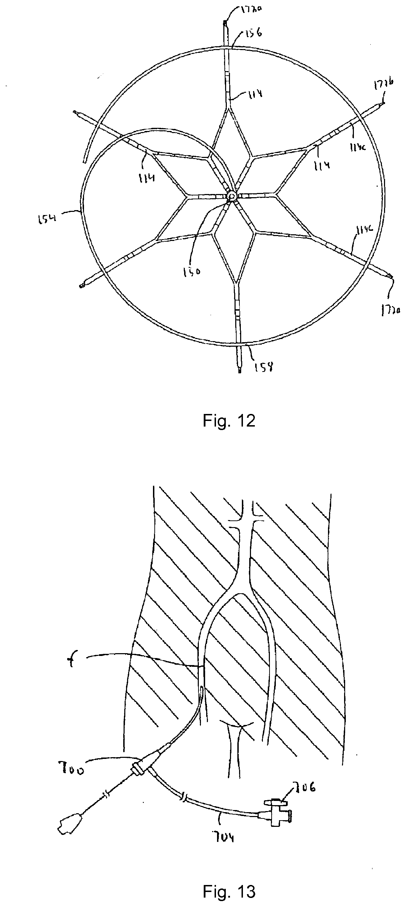

Figures 11 and 12 illustrate an alternate embodiment of the filter, designated by reference numeral 110. Filter 110 is identical to filter 10, except for the tubular portion and spacer, and therefore has been labeled with numerals in the "100" series corresponding to the double digit numbering of filter 10. Thus, filter 110 has struts 114, interconnecting struts 114a, 114b, hooks 172a, 172b, etc. and therefore for brevity these parts will not again be described.

Tubular portion 150 of filter 110 has a hook 192 identical to hook 92 of Figure 1. However, tubular portion 150 has a single spiral cutout 152, preferably formed by laser cutting, which forms a spiral spacer 154. The spiral spacer 154 has a shape memory position of that shown in Figures 11 and 12, extending radially from the tubular portion 150. When exposed from the sheath it unravels to move from a collapsed position substantially flush with the tubular portion 150 to its shape memory position forming an open loop starting at end 155 and wrapping over 360 degrees, terminating at edge 157. In this manner loop portions 156 and 158 are 180 degrees apart and the circular loop surfaces contact the inner wall of the vessel. It is also contemplated that the spacer can wrap a smaller or greater distance (degrees) than that shown and be oval or shapes other than circular. The loop can lie in a single plane or in multiple planes.

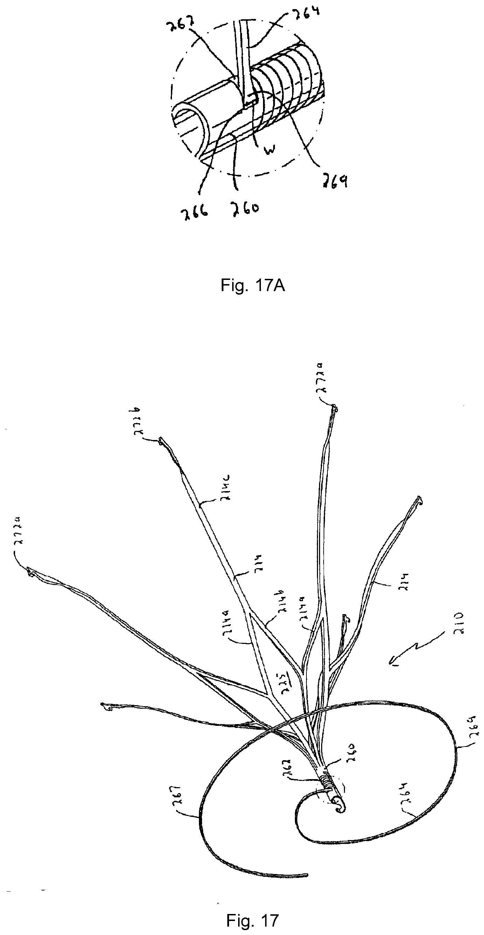

Figure 17 discloses an alternate embodiment of the filter which is identical to the filter shown in Figure 11 except for the spiral spacer 264. The filter 210 is labeled with reference numerals in the "200 series" corresponding to the "100 series" labeled parts of the Figure 11 embodiment and therefore has struts 214, hooks 272a, 272b, etc. The tubular portion and spacer, being different, have non-correlating reference numerals. More specifically, tubular portion 260 has a spiral cutout 262 to form a spacer 264. The distal (cranial) terminal end 266 of the cutout 262 has an increased width to form a spacer end 269 of increased width "w", shown in Figure 17A. This provides increased support for the spacer as it reduces stress at that part. Spiral spacer 264 loops around tubular portion 260 in a similar manner as spacer 154 of Figure 11, preferably wrapping over 360 degrees, although other degrees are contemplated. Opposed looped ends 267 and 269 are about 180° apart and the outer surfaces contact opposing sides of the vessel wall. As with spacers 154, the outer surfaces along the loop contact the vessel wall due to the circular configuration of the spacer 264.

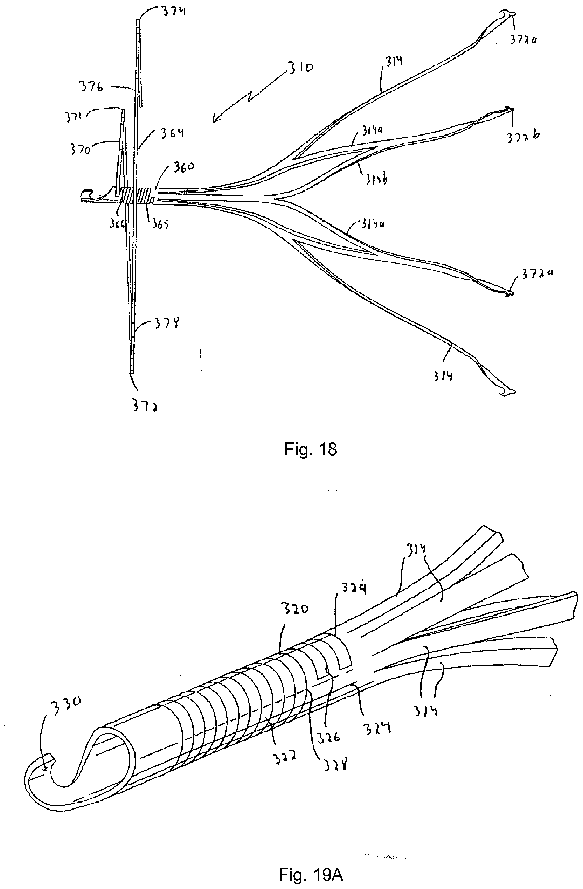

In the embodiment of Figure 18, the spacer 364 of filter 310 wraps around the tubular portion 360 in different planes, as opposed to the single plane of the embodiment of Figures 11 and 17. More specifically, in the expanded position, spacer 364 emerges from the distal (cranial) end 366 of cutout 365 and wraps at an angle toward the caudal end of the filter. Thus, as seen, the first end 372 of loop portion 370 lies in a plane proximal of the plane containing end 371 of loop portion 370 and distal of the plane containing the end 374 of loop portion 376. In other words, loop portion 378 lies, as viewed axially, between loops 370, 376. The remaining portions of the filter are identical to filter 210 of Figure 17 and are labeled with corresponding parts in the "300" series.

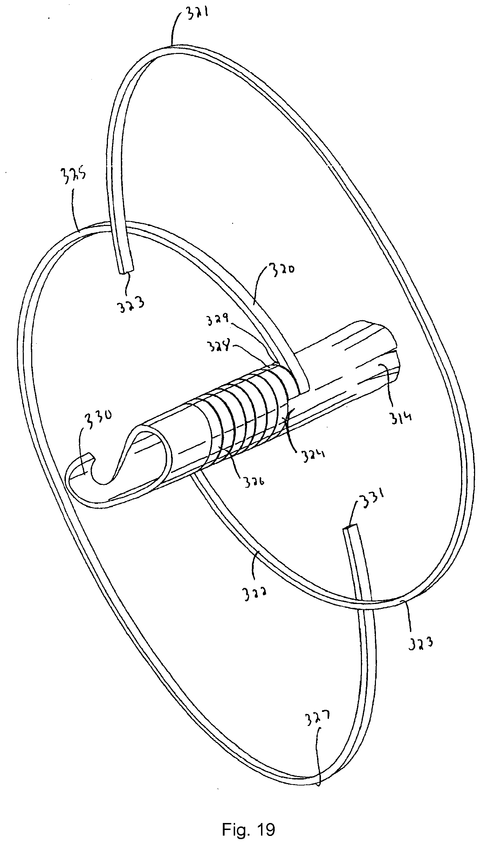

Figures 19 and 19A illustrate the cranial end of an alternate embodiment of the filter having two looped spacers extending radially from the tubular portion 324. In the collapsed position of Figure 19A, spacers 320, 322 are wrapped around tubular portion 324 so they are substantially flush with the wall of the tubular portion 324. The spacers 320, 322 are formed by two spiral cutouts 326, 328 formed in the wall of tubular portion 324. In the expanded position, spacer 320 emerges from the proximal (caudal) end 329 of cutout 328, extending in a substantially circular or spiral path around the tubular portion 324, preferably for about 300 degrees (although other degrees are contemplated), with surfaces 321, 323 about 180° apart contacting opposing surfaces of the vessel wall. Spacer loop 320 terminates at end 323 to form an open loop. Spacer 322 emerges from the distal (cranial) end of the cutout 326, wrapping around tubular portion 324 in the direction opposite of spacer 320. Similar to spacer 320, spacer 322 extends for about 300 degrees (although other degrees are contemplated), with opposing surfaces 325, 327 contacting opposite portions of the vessel wall. Spacer loop 322 terminates at end 331 to form an open loop. Hook 330 is preferably identical to hook 92 of the filter embodiment of Figure 1.

In the embodiment of Figure 20, open spacer loops 420 and 422 each start at a proximal end of cutout 426, with spacer 420 starting proximal of spacer 422. Cutout 426 has first cutout 426a and second cutout 426b, formed in an alternating pattern. Spacers 420, 422 lie in multiple planes, preferably wrap around tubular portion 424 in opposite directions, extending for about 300 degrees (although other degrees are contemplated) and have respective opposing surfaces 421, 423 and 425, 427, respectively for contacting opposing sides of the vessel wall. Spacers 420, 422 terminate in ends 430, 432.

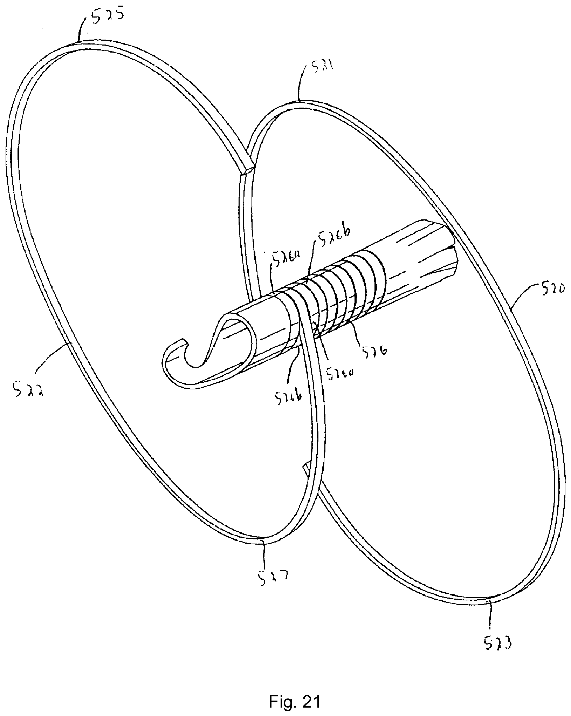

In the embodiment of Figure 21, open spacer loops 520, 522 are formed emerging from the distal end of cutouts 526a, 526b, with spacer 520 emerging distal of spacer 522. Spacers 520, 522 lie in different planes. Similar to loops 420, 422 of the embodiment of Figure 21, spacer 520 has opposing surfaces 521 and 523 and spacer 522 has opposing surfaces 525, 527. Loops 520, 522 lie in multiple planes.

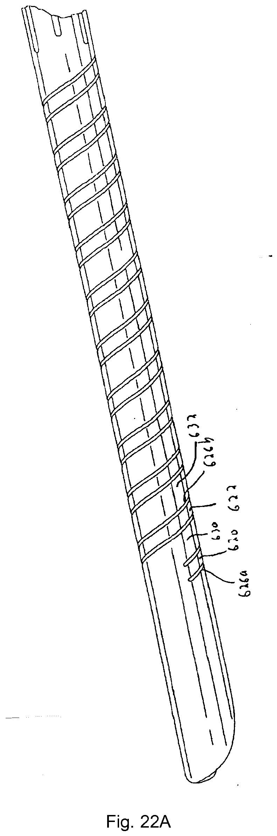

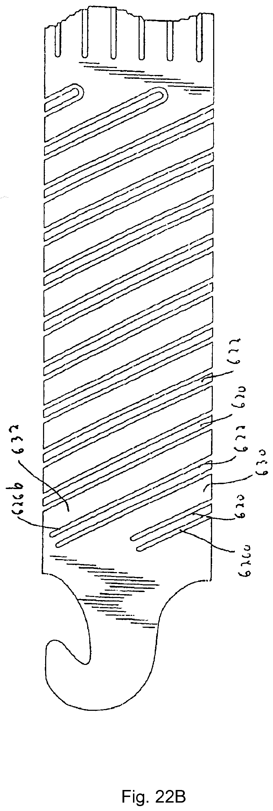

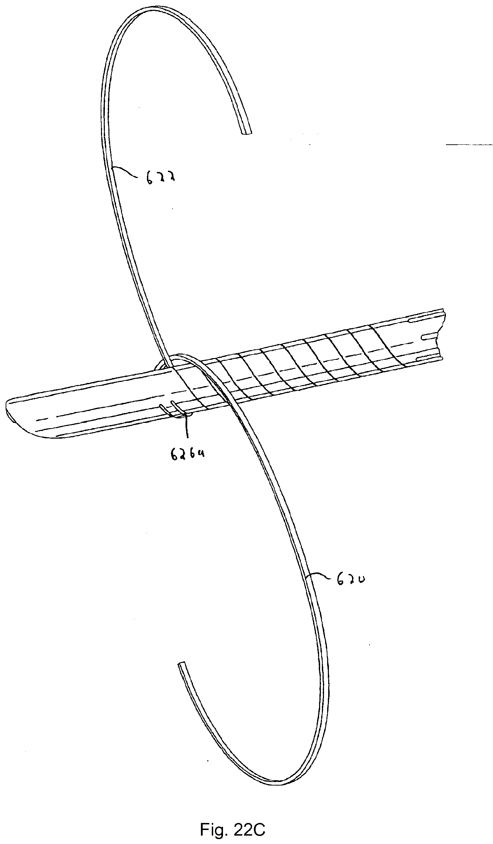

In the embodiment of Figure 22, open spacer loops 620, 622 extend from the distal end of cutout 626a, 626b. As shown, the cutouts are formed in an intertwined spiral fashion resulting in a spiral spacer when unraveled (expanded). The solid strip between cutout 626a is designated by reference numeral 630 and the solid strip between cutout 626b is designated by reference numeral 632.

To enable movement between an expanded and collapsed configuration, the filter of the embodiments described herein, as noted above, is preferably made of shape memory metal material, such as Nitinol, a nickel titanium alloy, and preferably manufactured from a laser cut tube. To facilitate passage of the filter through the lumen of the delivery sheath 700 (shown in Figure 13 in conjunction with the method of insertion) and into the vessel, cold saline is injected into the delivery sheath or catheter 700 and around the filter in its collapsed position within the delivery sheath 700. This shape memory material characteristically exhibits rigidity in the austenitic state and more flexibility in the martensitic state. The cold saline maintains the temperature dependent filter in a relatively softer condition as it is in the martensitic state within the sheath. This facilitates the exit of filter from the sheath 700 as frictional contact between the filter and the inner surface of the sheath would otherwise occur if the filter was maintained in a rigid, i.e. austenitic, condition.

Once ejected from the delivery sheath or catheter 700, the filter is no longer cooled and is exposed to the warmer body temperature, which causes the filter to return towards its austenitic memorized configuration.

In the placement (expanded) configuration, the filter moves towards its memorized position and the extent it returns to its fully memorized position will be dependent on the size of the vessel in which the filter is inserted. (The larger the vessel, the closer the filter comes to returning to it's fully memorized position). The extent of movement of the spacer(s) to its fully memorized position could also be limited by the size of the vessel.

The filter can be inserted through the jugular vein in the neck of the patient or through the femoral vein in the leg of the patient or the arm. The filters can also be placed in the superior vena cava.

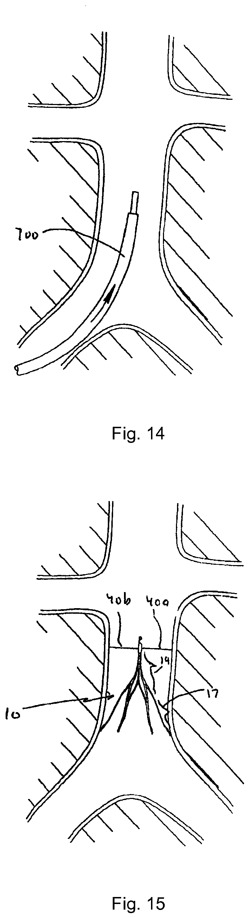

Figures 13-15 illustrate delivery and placement of the filter 10, by way of example, in the inferior vena cava. Delivery catheter or sheath 700 is inserted through the femoral vein "f" and advanced through the iliac arteries into the inferior vena cava. Delivery catheter 700 is withdrawn once the tip of the sheath is adjacent the structure so that withdrawal of the sheath would place the filter in the desired location of Figure 15. Tubing 704 and valve assembly 706 enable saline injection. Delivery catheter 700 is withdrawn to enable filter 10 to be warmed by body temperature to transition to the expanded placement configuration. The other filters described herein could be inserted in the same manner. Note it is implanted in the orientation such that filter section 19 is downstream of the flared section 17. This enables blood clots or other particles to be directed to the center of the filter section by the angled struts. Thus the direction of insertion, e.g. upstream or downstream direction, will determine how the filter is to be positioned in the delivery catheter.

The foregoing filters can be removed from access through the internal jugular or femoral vein. Various methods can be used to remove the filter such as those described in commonly assigned co-pending '429 application, including for example, slotted hooks, graspers, etc.

A recess or cutout is preferably provided at the tubular end portion to form a hook portion 90, as shown for example in Figures 7 and 9, having a curved hook 92 at the proximalmost end to receive a snare or other device for removal as described in the filter of the '429 application.

This hook 92 is configured to receive a retrieval snare or other retrieval device. A portion of the wall of the hook 90 is cut out to expose the annular interior surface 94. This annular interior surface 94 extends from radiused region 95 to proximalmost edge 96. The interior surface 94, for ease of explanation, can be considered to have an interior surface at the radiused region 95 and an interior surface 94b at the hook 92. The interior surface 94b accommodates a portion of a tubular snare sheath. That is, the outer wall of the snare sheath (tube) can partially fit within the cut out region. This enhances removal as the snare pulls the filter hook into collinear arrangement with the sheath tube as described and shown in Figures 13H-13N of the '429 application. The radiused region 95, spaced axially (distal) from the hook 92, includes a radiused or curved edge defined by radiused side walls 97a, 97c and top wall 97b. The angled side walls 97a, 97c form camming surfaces to direct the hook 90 and filter into the retrieval sheath.

When the filter is grasped by the retrieval device and pulled distally to disengage from the vessel walls, the spacers flex inwardly. This is shown for example in Figure 15, wherein spacers 40a, 40b of filter 10 flex in the direction of the arrow as the filter is pulled into retrieval sheath 800.

It should be appreciated, that the hook can be formed in other ways to provide an interior annular surface to function in a similar manner as surface 94, i.e. to receive the snare tube. When the filter is pulled into the retrieval sheath it is collapsed for removal.

Figure 9A illustrates an alternate embodiment of the hook portion 600 having an elongated hook 602 curving inwardly. This provides increased hooking area for the retrieval snare.

To facilitate removal of the filter from the vessel, cold saline can be injected onto the implanted filter to change the temperature of the filter to move it to a relatively softer condition to facilitate the filter being drawn into the retrieval sheath. That is, injection of cold saline will cause the filter to approach its martensitic state, bringing the filter to a more flexible condition. The flexible condition facilitates the collapse and withdrawal of the filter into the retrieval sheath by decreasing the frictional contact between the filter and the inner surface of the retrieval sheath.

A delivery system which can be used for the filter of the present invention which includes a filter cartridge, is shown and described in the '429 application.

While the above description contains many specifics, those specifics should not be construed as limitations on the scope of the disclosure, but merely as exemplifications of preferred embodiments thereof. For example, the foregoing filters can be inserted in other regions of the body. Also, the foregoing filters can be made of materials other than shape memory material. Those skilled in the art will envision many other possible variations that are within the scope of the claims appended hereto.

## Claims

1. A vessel filter comprising a body made from a single tube (11), the tube cut to create a plurality of elongated struts (14, 114, 214) forming a filter region (19) and a mounting region (17) having a greater transverse dimension than a transverse dimension of the filter region, the mounting region including a plurality of the elongated struts which terminate in vessel engaging hooks (72a, 72b, 172a, 172b, 272a, 272b), the hooks having a penetrating tip (76a, 76b) pointing in a direction toward the filter region, a plurality of teeth (79a, 79b) for engaging the vessel, and a heel (77a, 77b) extending beyond the teeth in a direction opposite the direction of the penetrating tip, **characterised in that** in a collapsed configuration of the filter the hooks (72a, 72b, 172a, 172b, 272a, 272b) extend substantially perpendicular to the respective strut (14, 114, 214) and in the plane thereof whereby the penetrating tip (76a, 76b) points in a first direction toward a cranial end of the filter and the teeth (79a, 79b) extend in a second direction substantially in a longitudinal axial direction of the filter so as to point toward the caudal end of the filter.

2. A vessel filter as claimed in claim 1, wherein the teeth (79a, 79b) are provided solely on the vessel engaging hooks (72a, 72b, 172a, 172b, 272a, 272b) which extend substantially perpendicular to the respective strut (14, 114, 214).

3. A vessel filter as claimed in claim 1 or 2, wherein the teeth (79a, 79b) are provided on a convexly curved surface of the vessel engaging hooks (72a, 72b, 172a, 172b, 272a, 272b).

4. A vessel filter as claimed in any preceding claim, wherein an end of the penetrating tip (76a, 76b) extends substantially parallel to the longitudinal axis of the strut.

5. A vessel filter as claimed in any preceding claim, wherein the heel (77a, 77b) of the hooks (72a, 72b, 172a, 172b, 272a, 272b) extends at an angle to the longitudinal axis of the respective struts (14, 114, 214) in the mounting region (17).

6. A vessel filter as claimed in any preceding claim, wherein the heels (77a, 77b) of adjacent vessel engaging hooks (72a, 72b, 172a, 172b, 272a, 272b) terminate axially spaced.

## Patentansprüche

1. Gefäßfilter, umfassend einen Körper, der aus einem einzigen Rohr (11) hergestellt ist, wobei das Rohr geschnitten ist, um eine Vielzahl von länglichen Streben (14, 114, 214) zu erzeugen, die eine Filterregion (19) bilden, und eine Montageregion (17), die eine größere Querabmessung als eine Querabmessung der Filterregion aufweist, wobei die Montageregion eine Vielzahl von länglichen Streben einschließt, die in Gefäß-eingreifenden Haken (72a, 72b, 172a, 172b, 272a, 272b) enden, wobei die Haken eine eindringende Spitze (76a, 76b) aufweisen, die in eine Richtung hin zur Filterregion zeigt, eine Vielzahl von Zähnen (79a, 79b) zum Eingriff des Gefäßes, und einen Absatz (77a, 77b), der sich über die Zähne hinaus in eine Richtung gegenüber der Richtung der eindringenden Spitze erstreckt, **dadurch gekennzeichnet, dass** in einer zusammengeklappten Konfiguration des Filters sich die Haken (72a, 72b, 172a, 172b, 272a, 272b) im Wesentlichen senkrecht zur jeweiligen Strebe (14, 114, 214) und in der Ebene davon erstrecken, wobei die eindringende Spitze (76a, 76b) in eine erste Richtung hin zu einem kranialen Ende des Filters zeigt und sich die Zähne (79a, 79b) in einer zweiten Richtung im Wesentlichen in einer längs-axialen Richtung des Filters erstrecken, um hin zum kaudalen Ende des Filters zu zeigen.

2. Gefäßfilter nach Anspruch 1, wobei die Zähne (79a, 79b) nur auf den Gefäß-eingreifenden Haken (72a, 72b, 172a, 172b, 272a, 272b) bereitgestellt sind, die sich im Wesentlichen senkrecht zur entsprechenden Strebe (14, 114, 214) erstrecken.

3. Gefäßfilter nach Anspruch 1 oder 2, wobei die Zähne (79a, 79b) nur auf einer konvex gekrümmten Fläche der Gefäß-eingreifenden Hakens (72a, 72b, 172a, 172b, 272a, 272b) bereitgestellt sind.

4. Gefäßfilter nach einem der vorhergehenden Ansprüche, wobei sich ein Ende der eindringenden Spitze (76a, 76b) im Wesentlichen parallel zur Längsachse der Strebe erstreckt.

5. Gefäßfilter nach einem der vorhergehenden Ansprüche, wobei sich der Absatz (77a, 77b) der Haken (72a, 72b, 172a, 172b, 272a, 272b) in einem Winkel zur Längsachse der entsprechenden Streben (14, 114, 214) in der Montageregion (17) erstreckt.

6. Gefäßfilter nach einem der vorhergehenden Ansprüche, wobei die Absätze (77a, 77b) von benachbarten Gefäß-eingreifenden Haken (72a, 72b, 172a, 172b, 272a, 272b) axial beabstandet enden.

## Revendications

1. Filtre pour vaisseau comprenant un corps constitué d'un simple tube (11), ce tube étant découpé pour créer une pluralité d'entretoises allongées (14, 114, 214) formant une région de filtrage (19) et une région de montage (17) présentant une dimension transversale supérieure à une dimension transversale de la région de filtrage, la région de montage comprenant une pluralité d'entretoises allongées qui se terminent par des crochets d'emboîtement avec le vaisseau (72a, 72bn 172a, 172b, 272a, 272b), ces crochets comprenant une pointe de pénétration (76a, 76b) pointant dans la direction de la région de filtrage, une pluralité de dents (79a, 79b) pour s'emboîter avec le vaisseau, et un talon (77a, 77b) s'étendant au-delà des dents dans une direction opposée à la direction de la pointe de pénétration, **caractérisé en ce que**, dans une configuration effondrée, du filtre, les crochets (72a, 72b, 172a, 172b, 272a, 272b) s'étendent globalement perpendiculairement à l'entretoise (14, 114, 214) respective et dans le plan de celle-ci, la pointe de pénétration (76a, 76b) pointant dans une première direction dune extrémité crânienne du filtre et les dents (79a, 79b) s'étendant dans une deuxième direction globalement dans une direction axiale longitudinale du filtre de façon à pointer en direction de l'extrémité caudale du filtre.

2. Filtre pour vaisseau selon la revendication 1, dans lequel les dents (79a, 79b) sont présentes uniquement sur les crochets d'emboîtement avec le vaisseau (72a, 72b, 172a, 172b, 272a, 272b) qui s'étendent globalement perpendiculairement à l'entretoise (14, 114, 214) respective.

3. Filtre pour vaisseau selon la revendication 1 ou 2, dans lequel les dents (79a, 79b) sont présentes sur une surface à courbure convexe sur les crochets d'emboîtement avec le vaisseau (72a, 72b, 172a, 172b, 272a, 272b).

4. Filtre pour vaisseau selon l'une des revendications précédentes, dans lequel une extrémité de la pointe de pénétration (76a, 76b) s'étend globalement parallèlement à l'axe longitudinal de l'entretoise.

5. Filtre pour vaisseau selon l'une des revendications précédentes, dans lequel le talon (77a, 77b) des crochets (72a, 72b, 172a, 172b, 272a, 272b) s'étend avec un angle par rapport à l'axe longitudinal des entretoises (14, 114, 214) respectives dans la région de montage (17).

6. Filtre pour vaisseau selon l'une des revendications précédentes, dans lequel les talons (77a, 77b) de crochets d'emboîtement avec le vaisseau (72a, 72b, 172a, 172b, 272a, 272b) adjacents se terminent de manière espacée axialement.
